Europäisches Patentamt

European Patent Office

Office européen des brevets

(19)

(11) **EP 0 808 464 B1**

(12) **EUROPÄISCHE PATENTSCHRIFT**

(45) Veröffentlichungstag und Bekanntmachung des Hinweises auf die Patenterteilung:
**11.04.2001 Patentblatt 2001/15**

(21) Anmeldenummer: **96901236.8**

(22) Anmeldetag: **01.02.1996**

(51) Int Cl.$^7$: **G01T 1/02**, G01T 1/161

(86) Internationale Anmeldenummer:
**PCT/DE96/00174**

(87) Internationale Veröffentlichungsnummer:
**WO 96/24859 (15.08.1996 Gazette 1996/37)**

(54) **MESSVERFAHREN UND SENSOR ZUR ON-LINE IN-VIVO BESTIMMUNG DER GEWEBEÄQUIVALENTEN DOSIS BEI DER STRAHLENTHERAPIE**

MEASURING PROCESS AND SENSOR FOR ON-LINE IN-VIVO DETERMINATION OF THE TISSUE-EQUIVALENT DOSE IN RADIOTHERAPY

PROCEDE DE MESURE ET CAPTEUR POUR LA DETERMINATION IN VIVO EN DIRECT DE LA DOSE EN EQUIVALENT-TISSU EN RADIOTHERAPIE

(84) Benannte Vertragsstaaten:
**AT CH DE FR GB LI SE**

(30) Priorität: **06.02.1995 DE 19503647**

(43) Veröffentlichungstag der Anmeldung:
**26.11.1997 Patentblatt 1997/48**

(73) Patentinhaber: **FORSCHUNGSZENTRUM JÜLICH GMBH**
**52425 Jülich (DE)**

(72) Erfinder:
• **HÄSING, Friedrich-Wolfgang**
**D-52428 Jülich (DE)**
• **BÜKER, Harald**
**D-52428 Jülich (DE)**

(56) Entgegenhaltungen:
**EP-A- 0 103 835       EP-A- 0 416 493**
**EP-A- 0 608 101       US-A- 5 014 708**

**Beschreibung**

Die Erfindung bezieht sich auf ein Verfahren zur in-vivo und on-line Bestimmung der gewebeäquivalenten Dosis bei der Strahlentherapie sowie auf eine Vorrichtung zur Durchführung des Verfahrens.

Es ist bekannt, eine annähernd gewebeäquivalente in-vivo Messung einer Dosis mit miniaturisierten Thermolumineszenz-Dosimetern in einigen Anwendungsfällen durchzuführen. Nachteilig ist jedoch die Beschränkung auf bestimmte Anwendungsfälle. Ferner steht das Meßergebnis erst nach aufwendiger Auswertung des Thermolumineszenz-Dosimeters frühestens eine Stunde nach Beendigung der Bestrahlung zur Verfügung.

Des weiteren gibt es zahlreiche Vorschläge und Versuche, die Dosis faseroptisch oder mit Halbleiterdosimetern in-vivo und on-line zu messen. (US 5,014,708 von Mai 1991; H. Büker et at, Fiber-Optic Radiation Dostmetne for Medical Application, SPIE, Vol 120 1, Optical Fibers in Medicine V, S. 419-429 (1990) , H. Büker et al., Physical Properties and Concepts for Applications of Attenuation-based Fiber-Optic Dosimeters for Medical Instrumentation, SPIE, Vol 1648, Fiber Optic Medical and Fluorescent Sensors and Applications, S. 63-70(1992)).

Aus EP 0 608 101 A3 ist ein Szintillationszähler mit zwei Szintillationsfasern als Sensor bekannt. Eine der Fasern ist gegen β-Strahlung abgeschirmt, so daß eine Faser β-und γ-Strahlung, die zweite Faser jedoch nur die γ-Strahlung detektiert. Durch Subtraktion der Ergebnisse kann die β-Stahlung bestimmt werden. Nachteilig kann mit dieser Vorrichtung jedoch keine gewebeäquivalente Dosis bestimmt werden.

Gemäß den Vorschlägen und Versuchen werden Sensoren eingesetzt, deren Material entweder eine vom Gewebe erheblich abweichende effektive Ordnungszahl aufweisen oder eine geringe Nachweisempfindlichkeit besitzen. Nachteilig ist jedoch, daß eine genaue Messung, insbesondere bei einer Photonenstrahlung, nicht möglich ist, da das Material des Sensors stets von dem des Gewebes abweicht und die absorbierte Dosis materialabhängig ist. Ferner ist prinzipiell keine Aussage über die Gewebetiefe möglich. Des weiteren ist nachteilig, in der Materialwahl beschränkt zu sein.

Es ist Aufgabe der Erfindung, die angeführten Nachteile durch Schaffung eines Meßverfahrens sowie einer Vorrichtung zur Durchführung des Verfahrens zur on-line und in-vivo Bestimmung der gewebeäquivalenten Dosis bei der Strahlentherapie zu überwinden.

Die Aufgabe wird durch ein Verfahren mit den Merkmalen des Anspruchs 1 gelöst.

im einzelnen kann das Verfahren nach Anspruch 1 wie folgt durchgeführt werden: Zunächst wird die Strahlendosis an einem Ort durch zumindest zwei Sensoren gemessen wird. Die Sensoren sind so zu wählen, daß sich innerhalb des Meßbereiches die Strahlendosis , proportional zum Meßsignal ändert. Ausreichend ist dabei eine durch eine Ausgleichsrechnung erzielte Proportionalität zwischen Meßsignal und Strahlendosis.

Mindestens zwei Meßsignale $S_1$ und $S_2$ der eingesetzten Sensoren 1 und 2 müssen eine voneinander abweichende Energieabhängigkeit der Nachweisempfindlichkeit für ionisierende Strahlung zeigen. Eine solche unterschiedliche Abhängigkeit ist bei optischen Sensoren mit unterschiedlichen effektiven Ordnungszahlen $Z_{eff}$ gegeben. Die unterschiedliche Anzeige kann jedoch auch durch eine unterschiedliche Abschirmung der Sensoren bewirkt sein.

Es wird zumindest ein Quotienten $Q_{12} = S_1 / S_2$ zweier gemessener Signale $S_1$ und $S_2$ dieser Sensoren 1 und 2 gebildet. Ein Signal $S_1$ bzw. $S_2$ kann mittels je einem Sensor 1 bzw. 2 oder aber beispielsweise als Mittelwert mehrerer Meßsignale von mehreren eingesetzten Sensoren, die die gleiche Abhängigkeit von der Nachweisempfindlichkeit für ionisierende Strahlung zeigen, ermittelt worden sein. Entscheidend ist, daß ein von der Dosis unabhängiger Wert bei der weiteren Auswertung verwendet wird. Alternativ zur einfachen Quotientenbildung kann daher auch mit Quotienten wie $(S_1 - S_2)/(S_1 + S_2)$ oder deren Kehrwerten fortgesetzt werden. Im folgenden wird die einfache Quotientenbildung $Q_{12}$ herangezogen.

Ferner sind die zu den berechneten Quotienten $Q_{12}$ gehörigen effektiven Gewebetiefen $d_g$ zu ermitteln. Dies geschieht anhand von Eichtabellen oder Eichkurven. Die benötigten Daten zur Erstellung dieser Tabellen bzw. Kurven sind durch Eichmessungen einer Dosisverteilung in Abhängigkeit von der geometrischen Tiefe $d_g$ in einem Phantom, also z. B. in einem Wasserbad oder einem PMMA-Phantom zu ermitteln. Diese geometrische Tiefe $d_g$ wird als effektive Gewebetiefe bezeichnet, da die geometrischen liefe während der Messung grundsätzlich von der Tiefe während der Eichmessung abweicht.

Aus den ermittelten effektiven Gewebetiefen $d_g$ sind die von $d_g$ abhängigen Kalibrierfaktoren $K_1(d_g)$ und $K_2(d_g)$ der Sensoren $S_1$ und $S_2$ zu bestimmen. Die Kalibrierfaktoren können experimentell im Phantom gegen eine Ionisationskammer gemäß

$$S_i(d_g) = K_i(d_g)^{-1} * D \qquad \text{mit i = i, 2, ..}$$

tiefenabhängig bestimmt werden.

Die gewebeäquivalente Dosis D wird dann gemäß

$$D = \left( \sum_{i=1}^{N} K_i(d_g) * S_i \right) \ / \ N$$

berechnet, wobei N die Anzahl der Kalibrierfaktoren darstellt.

Vorteile des Verfahrens sind: Das Material des Sensors muß nicht auf das Gewebe abgestimmt sein und kann daher frei gewählt werden. Die gewebeäquivalente Dosis wird sehr genau ermittelt. Außerdem steht mit der effektiven Gewebetiefe ein Maß für Tiefe im Gewebe, in der gemessen worden ist, zur Verfügung.

Das Verfahren gemäß rückbezogenem Verfahrensanspruch stellt eine vorteilhafte Ausführungsform dar. Wird ein Signal $S_1$ bzw. $S_2$ zweimal durch zwei gleiche Sensoren gemessen, so kann auch anstelle eines Einzelwertes eine entsprechende Mittelwertbildung aus diesen beiden Signalen für die weitere Berechnung herangezogen werden.

Eine Meßeinrichtung zur verfahrensgemäßen Bestimmung einer gewebeäquivalenten Strahlendosis weist mindestens zwei Sensoren auf, die eine voneinander abweichende Energieabhängigkeit der Nachweisempfindlichkeit für ionisierende Strahlung zeigen.

Unter einem Sensor ist jedes Bauteil zu verstehen, welches einer seiner physikalischen, chemischen oder technischen Eigenschaften bei Bestrahlung ändert und diese Änderung als Maß für die durch die Strahlung bewirkte Strahlendosis geeignet ist. Die Änderung ist geeignet, wenn mit zunehmender Strahlendosis eine kontinuierliche Änderung der physikalischen, chemischen oder technischen Eigenschaft einhergeht.

Beispiele für derartige Bauteile sind mikro- oder faseroptische Sensoren, z. B. bekannt aus DE 3929294 Al oder DE 32 34 900 Al. Unter mikrooptischem Sensor ist ein Sensor mit einem Durchmesser kleiner als 1 mm zu verstehen.

Beispiel für eine derartige Änderung ist die Änderung einer optischen Eigenschaft, wie induzierte Dämpfung, Szintillation oder Fluoreszenz, bekannt aus DE 3929294A1. Gemäß DE 3929294 A1 werden Sensoren über faseroptische, strahlungsresistente Übertragungsleitungen bzw. -fasern mit einer Meß- und Auswerteelektronik verbunden. Die Meßsignale werden durch die Elektronik angezeigt. Bestehen die Sensoren aus mikromechanischen Bauelementen, so kann jeder einzelne Sensor in je einem Lumen eines mehrlumigen Katheters angeordnet sein.

Zwei Sensoren zeigen eine voneinander abweichende Energieabhängigkeit der Nachweisempfindlichkeit für ionisierende Strahlung, wenn sich die Meßsignale unterschiedlich ändern. Diese Änderung kann durch die Bauart bedingt sein, z. B. durch eine unterschiedliche effektive Ordnungszahl bei optischen Sensoren oder aber infolge von unterschiedlichen Abschirmungen der Sensoren.

Bei einer vorteilhaften Ausführungsform ist die Meßeinrichtung rotationssymmetrisch bezüglich ihrer Sensoren aufgebaut und besteht aus mindestens drei Sensoren, von denen mindestens zwei die gleiche Energieabhängigkeit der Nachweisempfindlichkeit für ionisierende Strahlung zeigen.

Sind zwei Sensoren baugleich, z. B. optische Sensoren mit gleicher effektiver Ordnungszahl, so zeigen die Sensoren eine gleiche Energieabhängigkeit der Nachweisempfindlichkeit. Unterschiede von Meßsignalen, die im Rahmen von Meßungenauigkeiten liegen oder die produktionsbedingt sind, sind unerheblich. Die Rotationssymmetrie bezieht sich auf die Mittelachse des Aufbaus, entlang der die Sensoren z. B. in ein Gewebe eingeführt werden. Aufgrund des rotationssymmetrischen Aufbaus ist die Einrichtung unempfindlich gegenüber einer Rotation um diese Mittelachse.

Es zeigen

Fig. 1: planares Trägerelement zur Ankopplung mehrerer faseroptischer strahlungsempfindlicher Sensoren
Fig. 2: Metallkapillare als Trägerelement zur Ankopplung von faseroptischen strahlungsempfindlichen Sensoren
Fig. 3: Aufbau mit einem szintillierendem und einem die strahlungsinduzierte Dämpfung messenden faseroptischen Sensor
Fig. 4: drei strahlungsempfindliche Sensoren in einem mehrlumigen Katheter
Fig. 5 a, b: Längs- und Querschnitt eines Aufbaus mit drei strahlungsempfindlichen Sensoren
Fig. 6: effektive Gewebetiefe in Abhängigkeit von dem Verhältnis der Signàle zweier Sensoren mit unterschiedlicher effektiver Ordnungszahl
Fig. 7: Abhängigkeit der Kalibrierfaktoren zweier Sensoren mit unterschiedlicher effektiver Ordnungszahl von der effektiven Gewebetiefe
Fig. 8: Dosismessungen in Abhängigkeit von der Gewebetiefe

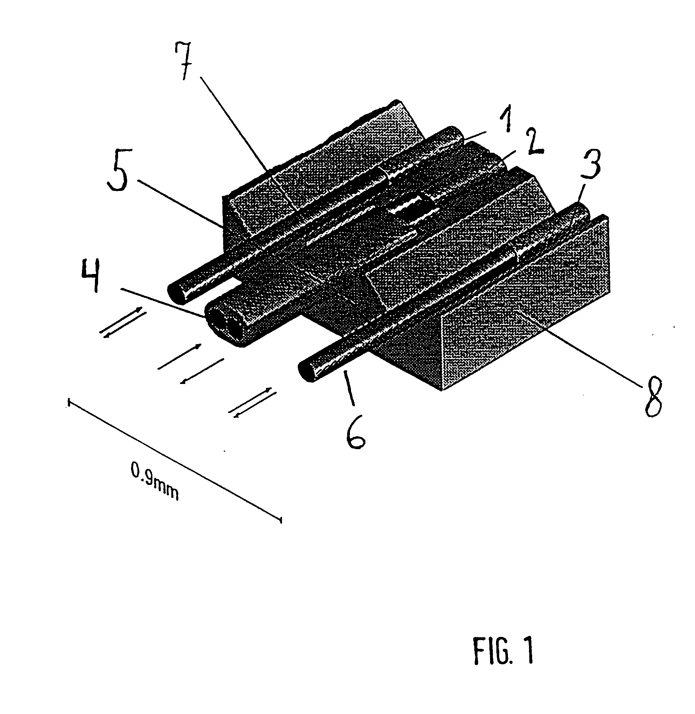

Figur 1 zeigt einen rotationssymmetrischen Aufbau mit drei strahlungsempfindlichen Sensorfasern 1, 2 und 3. Die mittlere Sensorfaser 2 besteht aus einer PbO-Faser mit 60 Gew% Bleioxyd. Diese ist an eine strahlungsunempfindliche Zwillingsfaser 4, bestehend aus zwei Quarzfasern (Hard - Clad - Fasern mit hoher numerischer Appertur) in einer gemeinsamen Ummantelung 5, gekoppelt.
Die Sensorfasern 1 und 3 sind Ge-P dotierte Gradienten-Index-Fasern (Germanium ungefähr 26 Gew%, Phosphor ungefähr 4 Gew%), die an den handelsüblichen, strahlungsharten Nachrichtenfasern 6 und 7 (z. B. AT&T-Rad Hard 3A) angespleißt sind. Die Sensorfasern 1, 2 und 3 sind im planaren Substrat 8 rotationssymmetrisch zur Längsachse der mittleren Faser 2 eingebettet. Das Substrat 8 besteht aus Metall, Glas oder Silizium. Insgesamt ist der planare Aufbau ungefähr 0,9 mm breit.

Die Zwillingsfaser wird als Übertragungsfaser eingesetzt, um störende Fresnel-Reflexionen beim Auslesen der strahlungsinduzierten Lichtschwächung zu vermeiden. Die rotationssymmetrische Anordnung der Sensoren bewirkt eine Unempfindlichkeit der Meßeinrichtung gegenüber einer Rotation um die Längsachse des mittleren Sensors 2 im Strahlungsfeld. Die Verwendung zweier Germanium-Phosphor-Sensoren verbessert zudem das Signal-Rauschverhältnis des Meßsignals beim Auslesen der Germanium-Phosphor-Sensoren 1 und 3.

Werden die Sensorfasern 1,2 und 3 bestrahlt, so nimmt die Lichtdämpfung in den Sensorfasern mit zunehmender Dosis zu. Die Dämpfung ist folglich ein Maß für die Strahlendosis. Die Abhängigkeit zwischen Dämpfung und Dosis bei der PbO-Faser unterscheidet sich von der Abhängigkeit bei den Ge-P-dotierten Fasern 1 und 3 aufgrund unterschiedlicher effektiver Ordnungszahlen.

Die Enden der Sensoren sind verspiegelt, die den Enden gegenüberliegen, an die die Übertragungsfasern 4, 6 und 7 gekoppelt sind. Die Verspiegelung dient der Lichtreflexion. Von der Meß- und Auswerteelektronik ausgehend gelangt Licht über die Übertragungsfasern 4, 6 und 7 in die Sensoren. Das Licht wird an den verspiegelten Enden reflektiert und wird so zurück zur Elektronik gelenkt. Die Laufrichtung des Lichtes wird in der Fig. 1 durch die sechs, parallel verlaufenden Pfeile (vor den Übertragungsleitern) verdeutlicht. Die Elektronik registriert die Änderung der Dämpfung und zeigt diese Änderung als Maß für die Dosis an.

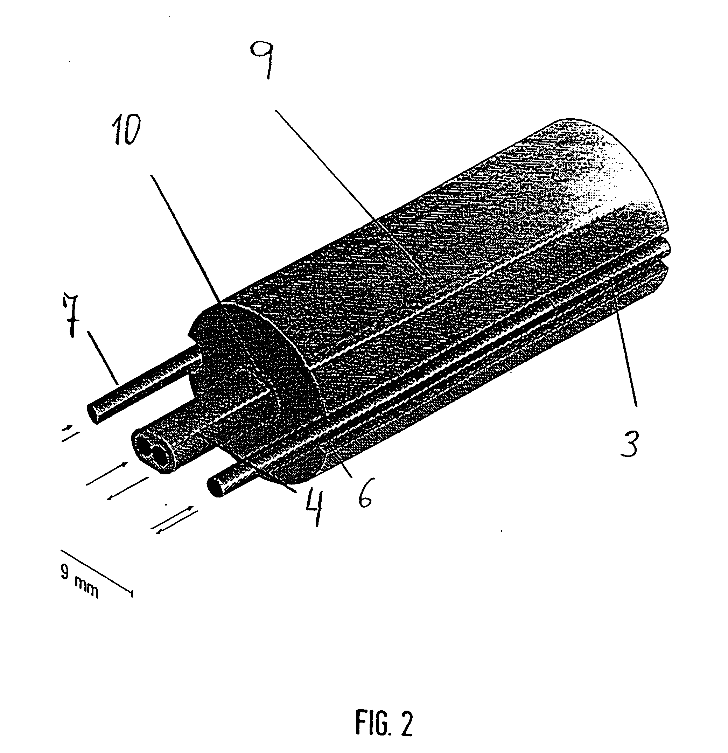

Fig. 2 zeigt im Prinzip den Aufbau aus Fig. 1. Einziger Unterschied ist die Einbettung in die VA-Kapillare 9 anstelle des planaren Substrats aus Fig. 1. Die Zwillingsfaser ist mittels Epoxy-Kleber 10 fixiert.

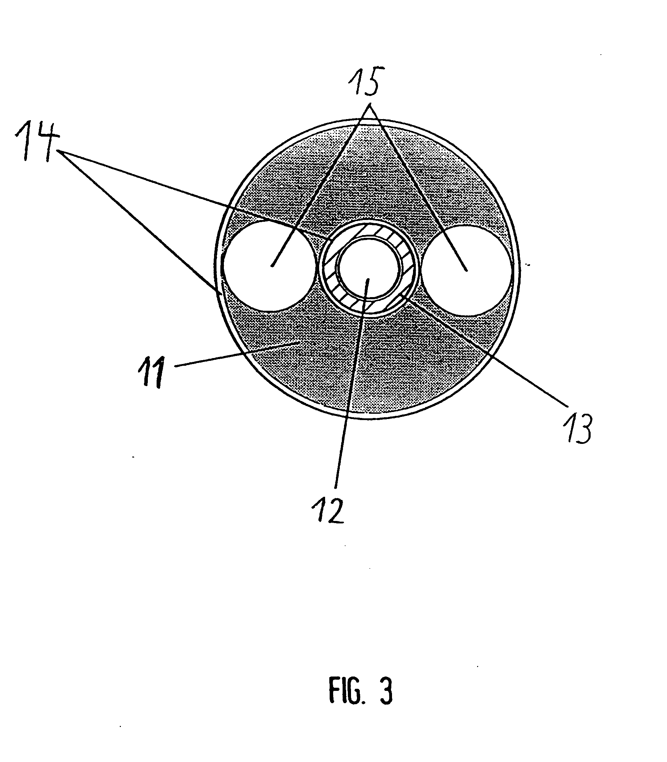

Der in Fig. 3 im Querschnitt gezeigte Aufbau besteht aus einem szintillierenden NaI-Kristall 11 als ersten Sensor und einer PbO-Faser 12 als zweiten Sensor. Der NaI-Kristall 11 dient gleichzeitig als Trägerelement für die PbO-Faser, die zudem von der VA-Kapillare 13 umhüllt wird. Zur Erhöhung der Lichtausbeute des szintillierenden Elementes sind seine Endflächen verspiegelt und seine innere und äußere Mantelfläche 14 mit einem lichtstreuenden Material geringer Absorption, z. B. Bariumsulfat oder Titandioxyd, beschichtet. Die Lichtauskopplung aus dem szintillierendem Sensor 11 erfolgt durch ein oder mehrere Fenster 15, vor denen Lichtwellenleiter fixiert sind. Die Ankopplung des PbO-Sensors erfolgt wiederum mittels einer Zwillingsfaser.

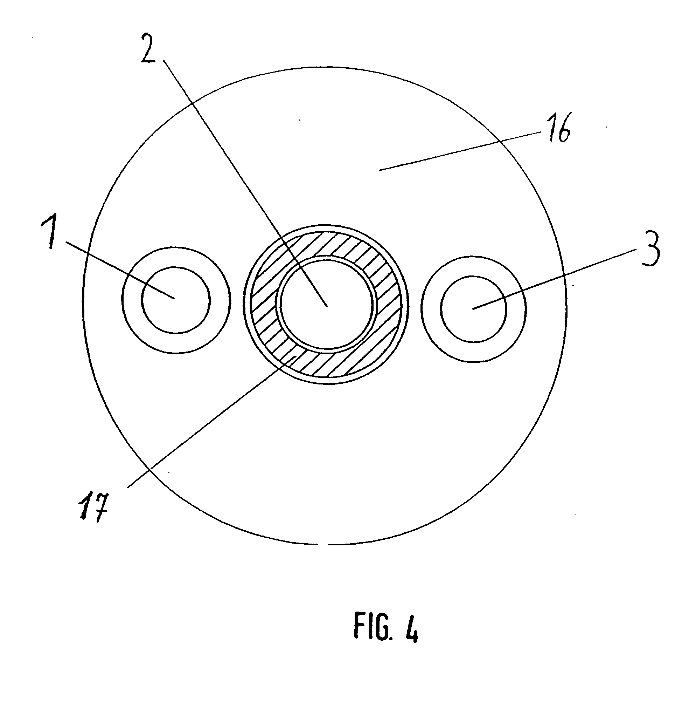

In Fig. 4 wird ein Aufbau analog Fig. 1 und 2 dargestellt. Diesmal wird jedoch ein dreilumiger, im Querschnitt dargestellter Katheterschlauch 16 zur Positionierung der Sensoren 1,2 und 3 eingesetzt. Die PbO-Faser 2 ist von einer Stahlkapillare 17 umhüllt. Die Stahlkapillare 17 dient der Ankopplung einer Zwillingsfaser.

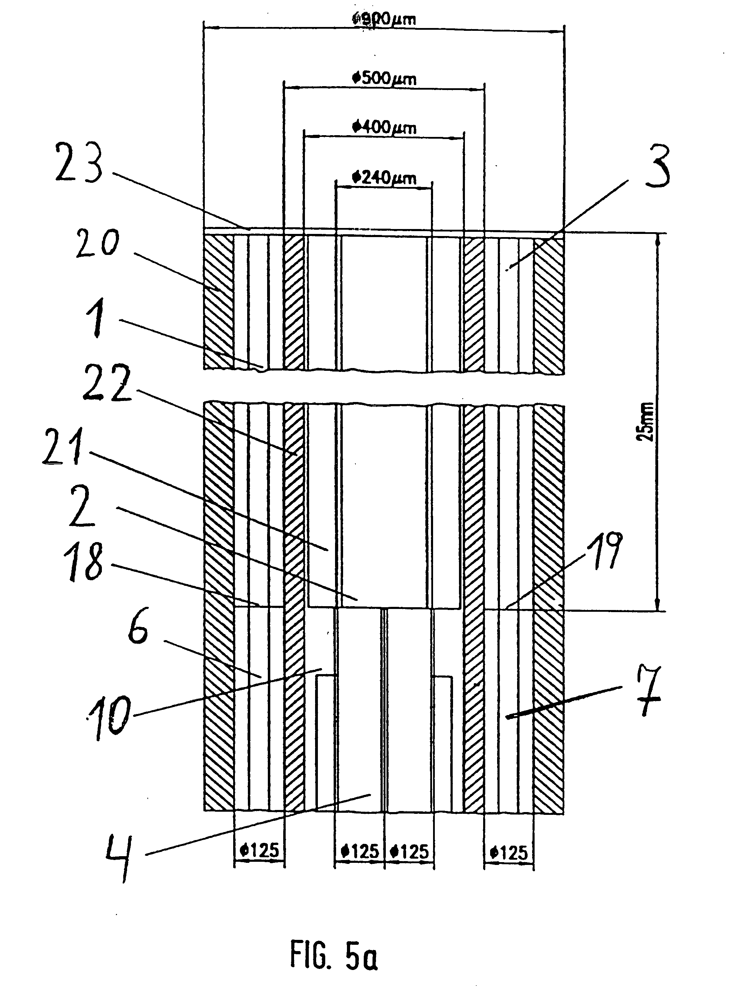

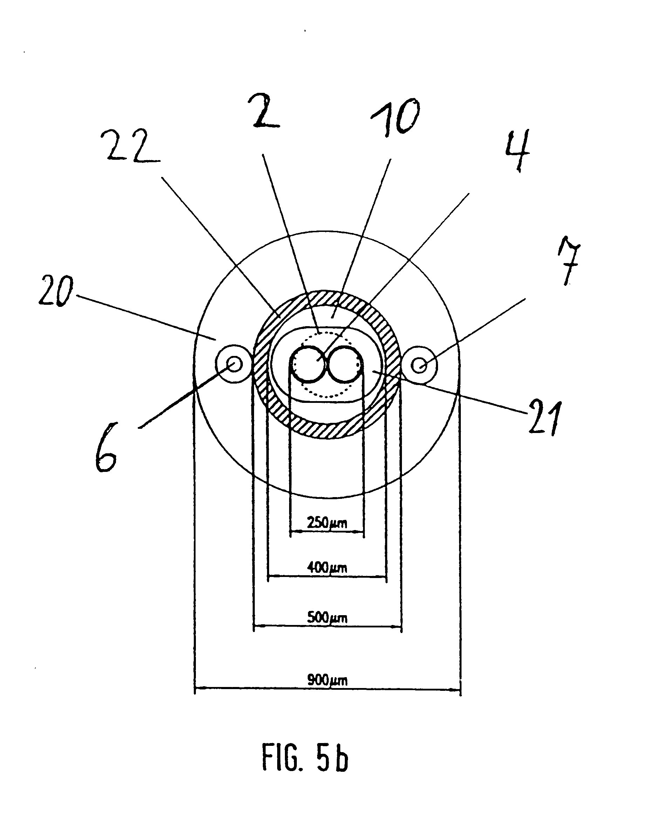

In Fig. 5 a, b ist ein weiteres Ausführungsbeispiel mit drei Sensorfasern 1, 2 und 3 analog Fig. 1, 2 oder 3 im Längs- (Fig. 5a) und Querschnitt (Fig. 5 b) dargestellt. Die Ge-P-dotierten Gradientenindexfasern 1 und 3 sind an den Stellen 18 und 19 an strahlungsharte Übertragungsfasern 6 und 7 angespleißt und werden durch die biokompatible Vergußmasse 20 geschützt. Die PbO-Faser 2 ist zunächst von einer Ummantelung 21 und dann von einer Metallkapillare 22 umgeben. Der Al-Spiegel 23 dient wie erläutert der Lichtreflexion.

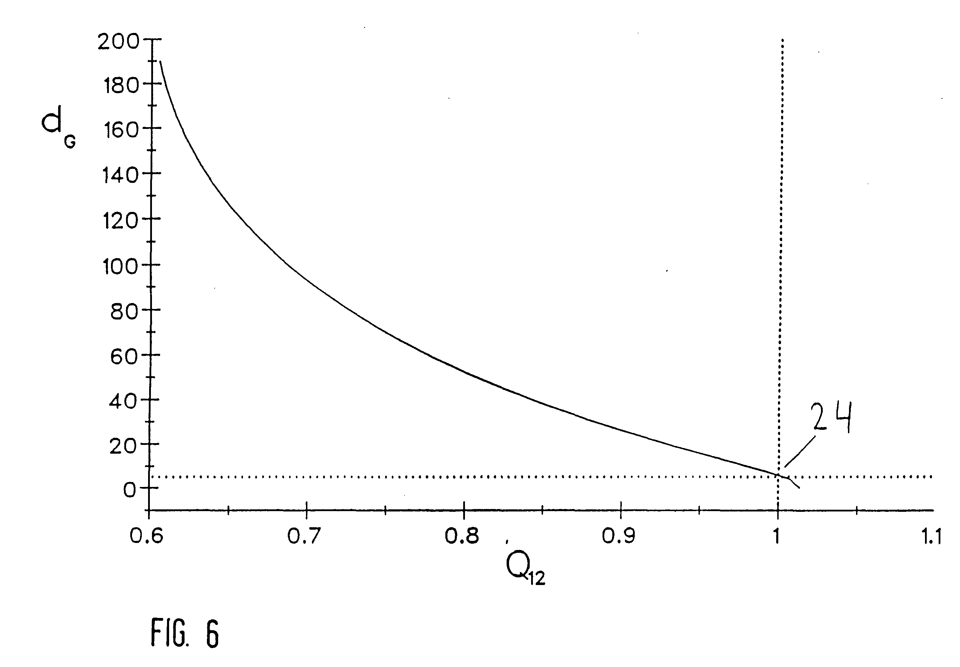

Mit allen dargestellten Sensoren kann das Verfahren gemäß Anspruch 1 durchgeführt werden. Die in Fig. 6, 7 und 8 dargestellten Meßergebnisse wurden mit einem Aufbau gemäß Fig. 5 a, b erzielt. Die gewebeäquivalente Dosis wurde dabei gemäß der Beschreibung des Verfahrens ermittelt.

Fig. 6 zeigt die effektive Gewebetiefe $d_g$ in Abhängigkeit von dem Quotienten $Q_{12}$ der beiden Dosisanzeigen $S_1$ und $S_2$, wenn der Doppelsensor im Dosismaximum der Tiefendosisverteilung kalibriert wurde. Das eine Signal $S_1$ stellt den Mittelwert der beiden von den Ge-P-Sensoren 1 und 3 stammenden Signale dar. Der Kalibrierungspunkt befindet sich im Schnittpunkt 24 der punktierten Linien.

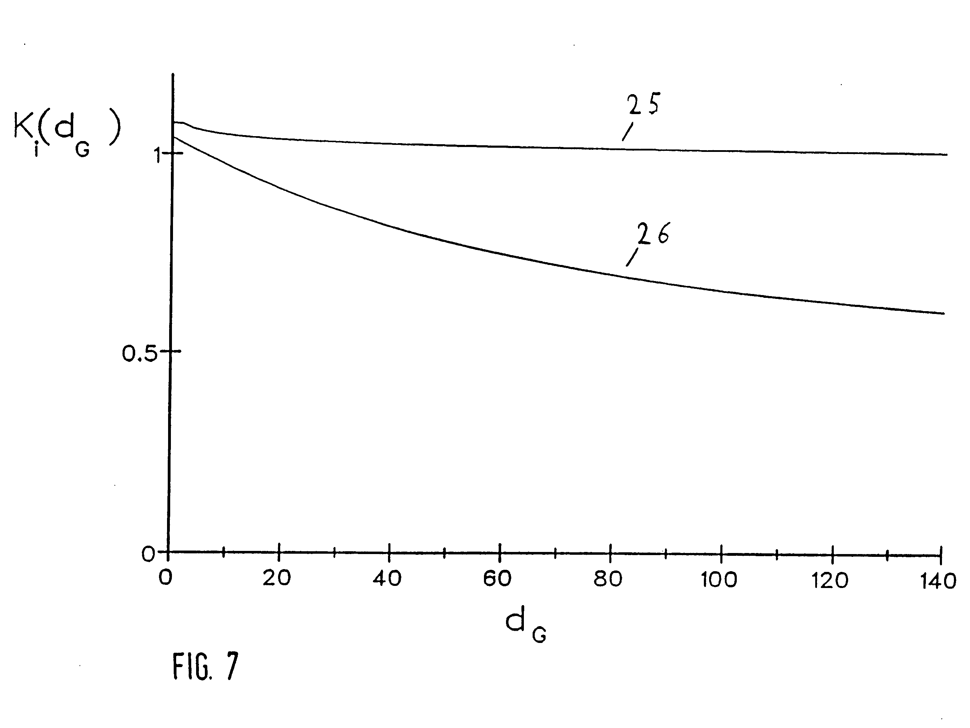

In Fig. 7 ist die Abhängigkeit der Kalibrierfaktoren $K_i(d_g)$ der Sensorelemente 1 und 2 mit unterschiedlicher effektiver Ordnungszahl von der effektiven Gewebetiefe dg dargestellt (i= 1, 2). Kurve 25 ist mit dem Sensor 1 und Kurve 26 mit dem Sensor 2 aufgenommen worden.

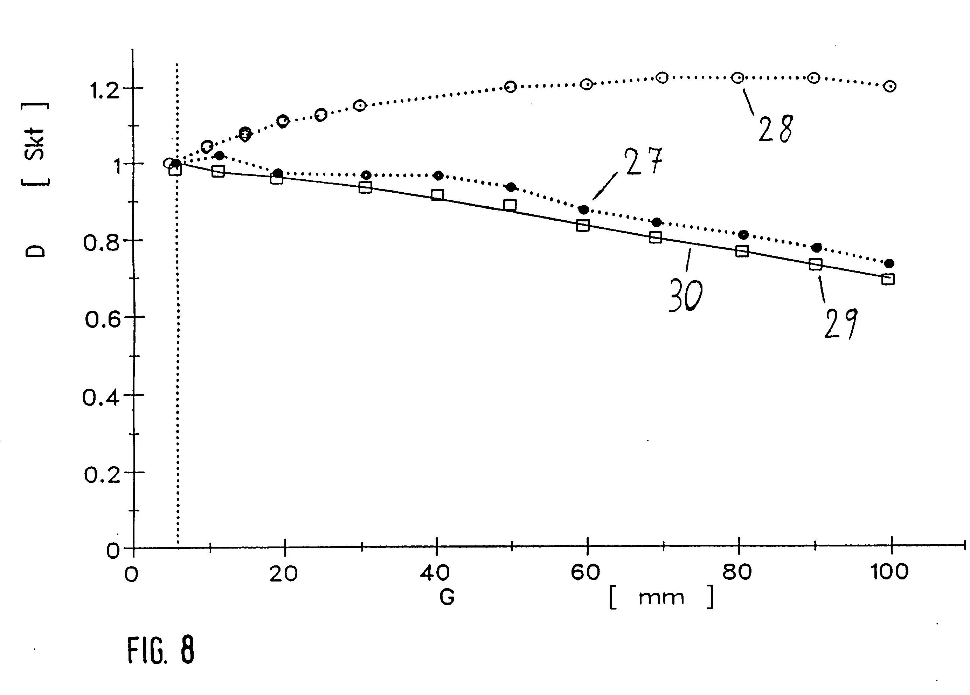

Fig. 8 zeigt Tiefendosismessungen mit dem Ge-P-Sensor 1 (kreisförmige Punkte 27) sowie dem PbO-Sensor 2 (Kreise 28) bei Bestrahlungen mit einer Co60-Strahlenquelle. Aufgetragen ist die Dosis D (Skt = Skalenteile) gegen die geometrische Gewebetiefe G (mm = Millimeter). Die senkrecht punktierte Linie kennzeichnet die Tiefe, in der kalibriert wurde. Ge-P-Sensoren und PbO-Sensoren zeigen eine voneinander abweichende Energieabhängigkeit der Nachweisempfindlichkeit für ionisierende Strahlung, wie die voneinander abweichenden Meßpunkte 27 verglichen mit den Meßpunkten 28 verdeutlichen. Die Quadrate 29 stellen das verfahrensgemäß ermittelte Ergebnis dar. Ein Vergleich der ermittelten Werte 29 mit der, mittels durchgezogener Linie 30 dargestellten Dosisanzeige einer Ionisationskammer zeigt, daß das Verfahren ein nahezu gewebeäquivalentes Ergebnis liefert und insbesondere genauer als das durch den PbO-Sensor gemessene Resultat ist.

## EP 0 808 464 B1

**Patentansprüche**

1. Verfahren zur in-vivo und on-line Bestimmung einer gewebeäquivalenten Dosis mittels Sensoren bei der Strahlungstherapie, mit den Schritten

   - eine Strahlung wird durch zumindest zwei Sensoren (1, 2), welche eine voneinander abweichende Energieabhängigkeit der Nachweisempfindlichkeit für ionisierende Strahlung aufgrund einer unterschiedlichen effektiven Ordnungszahl zeigen und am selben Ort liegen im Gewebe liegen, gleichzeitig gemessen;

   - aus den durch die zwei Sensoren (1, 2) gemessenen Meßsignale wird zumindest ein dosisunabhängiger Quotient gebildet;

   - die zu dem aus den Meßsignalen gebildeten Quotienten gehörige effektive Gewebetiefe $d_g$ wird anhand einer Kalibrierkurve bestimmt;

   - die von der effektiven Gewebetiefe $d_g$ abhängigen Kalibrationsfaktoren $K_i(d_g)$ der Sensoren (1, 2) werden anhand einer weiteren Kalibrierkurve bestimmt;

   - hieraus wird die gewebeäquivalente Dosis D gemäß

$$D = \left( \sum_{i=1}^{N} K_i(d_g) * S_i \right) / N,$$

   wobei N die Anzahl der Sensoren darstellt, ermittelt.

2. Meßvorrichtung zur Bestimmung einer Strahlendosis nach einem Verfahren nach Anspruch 1

   - mit wenigstens zwei Sensoren für die Messung der Strahlung, wobei die Sensoren eine voneinander abweichende Energieabhängigkeit der Nachweisempfindlichkeit für ionisierende Strahlung aufgrund einer unterschiedlich effektiven Ordnungszahl aufweisen;
   - mit einem Speicherelement, in dem die Abhängigkeit des Quotienten von effektiven Gewebetiefen anhand von Kalibriertabellen oder Kalibrationskurven und in dem die Abhängigkeit von Kalibrierfaktoren von der effektiven Gewebetiefe gespeichert sind;
   - und mit einer Meß- und Auswerteelektronik, die so beschaffen ist, daß zumindest ein Quotient der aus den durch die zwei Sensoren ermittelten Meßsignalen S1 und S2 gebildet wird und unter Verwendung des bzw. der Quotienten die gewebeäquivalente Dosis ermittelt wird.

3. Meßvorrichtung zur Bestimmung einer Strahlendosis nach vorhergehendem Anspruch mit drei parallel in einer Ebene angeordneten, faseroptischen Sensoren, wobei die äußeren Sensoren die gleiche Energieabhängigkeit und der mittlere Sensor eine hiervon abweichende Energieabhängigkeit der Nachweisempfindlichkeit für ionisierende Strahlung zeigen und die äußeren Sensoren in identischer Entfernung zum mittleren Sensor angeordnet sind.

**Claims**

1. A method for the in-vivo and on-line determination of a tissue-equivalent dose by means of sensors during radiation therapy, comprising the following steps:

   - radiation is measured simultaneously by at least two sensors (1, 2) which have energy-dependences of their detection sensitivities for ionising radiation which differ from each other due to their different effective atomic numbers, and which are situated at the same place in the tissue;

   - at least one dose-independent quotient is formed from the measuring signals which are measured by the two sensors (1, 2);

- the effective tissue depth $d_g$ which is associated with the quotient formed from the measuring signals is determined with the aid of a calibration curve;

- the calibration factors $K_i(d_g)$ of the sensors (1, 2), which calibration factors are dependent on the effective tissue depth $d_g$, are determined with the aid of a further calibration curve;

- the tissue-equivalent dose D, which is given by

$$D = \left( \sum_{i=1}^{N} K_i(d_g) * S_i \right) / N,$$

wherein N constitutes the number of sensors, is determined therefrom.

2. A measuring device for determining a radiation dose by a method according to claim 1,

   - having at least two sensors for measuring the radiation, wherein the sensors have energy-dependences of their detection sensitivities for ionising radiation which differ from each other due to their different effective atomic numbers;
   - having a memory element in which the dependence of the quotient on the effective tissue depth is stored with the aid of calibration tables or calibration curves, and in which the dependence of calibration factors on the effective tissue depth is stored;
   - and having a measuring and evaluation electronics unit which is designed so that at least one quotient is formed from the measuring signals S1 and S2 which are determined by the two sensors, and the tissue-equivalent dose is determined using the quotient or quotients.

3. A measuring apparatus for determining a radiation dose according to the proceeding claim, having three fibre-optic sensors which are disposed in parallel in a plane, wherein the outer sensors have same energy-dependences of their detection sensitivities for ionising radiation and the middle sensor has an energy-dependence of its detection sensitivity for ionising radiation which differs therefrom, and the outer sensors are disposed at an identical distance from the middle sensor.

**Revendications**

1. Procédé pour déterminer in vivo et en ligne une dose équivalente de tissu à l'aide de capteurs lors d'une radiothérapie, comportant les étapes suivantes :

   - un rayonnement est mesuré simultanément par au moins deux capteurs (1, 2), qui ont une sensibilité de détection vis-à-vis du rayonnement ionisant qui, en raison d'un numéro atomique effectif différent, varie de deux manières différentes en fonction de l'énergie, les deux capteurs se trouvant au même point dans le tissu ;
   - à partir des signaux de mesure mesurés par les deux capteurs (1, 2), on forme au moins un quotient indépendant de la dose ;
   - on détermine à l'aide d'une courbe d'étalonnage la profondeur effective de tissu $d_g$ appartenant au quotient formé à partir des signaux de mesure ;
   - on détermine à l'aide d'une autre courbe d'étalonnage les facteurs d'étalonnage $K_i(d_g)$ des capteurs (1, 2), qui dépendent de la profondeur effective de tissu $d_g$ ;
   - à partir d'eux, on détermine la dose équivalente de tissu D par la formule suivante :

$$D = \left( \sum_{i=1}^{N} K_i(d_g) * S_i \right) / N,$$

dans laquelle N est le nombre des capteurs.

2. Appareil de mesure pour déterminer une dose de rayonnement par un procédé selon la revendication 1,

- comportant au moins deux capteurs pour la mesure du rayonnement, la sensibilité de détection des capteurs vis-à-vis d'un rayonnement ionisant variant de deux manières différentes en fonction de l'énergie, en raison d'un numéro atomique effectif différent ;
- comportant un élément de mémoire, dans lequel la relation entre les quotients et les profondeurs effectives de tissu est mémorisée à l'aide de tableaux d'étalonnage ou de courbes d'étalonnage, et dans lequel est mémorisée la relation entre les facteurs d'étalonnage et la profondeur effective de tissu ;
- et comportant une électronique de mesure et d'évaluation, qui est conçue de telle sorte que soit formé un quotient des signaux de mesure $S_1$ et $S_2$ déterminés par les deux capteurs, ce quotient étant déterminé par utilisation du ou des quotients de la dose équivalente de tissu.

3. Appareil de mesure pour déterminer une dose de rayonnement selon la revendication ci-dessus, comportant trois capteurs à fibres optiques, disposés en parallèle dans un plan, la sensibilité de détection des capteurs extérieurs vis-à-vis d'un rayonnement ionisant variant de la même manière en fonction de l'énergie, et celle du capteur central variant d'une manière différent en fonction de l'énergie, les capteurs extérieurs étant disposés à une distance identique du capteur central.

0.9mm

FIG. 1

FIG. 2

FIG. 3

FIG. 4

FIG. 5a

FIG. 5b

FIG. 6

EP 0 808 464 B1

FIG. 7

FIG. 8

28

27

29

30

G [ mm ]

D [ Skt ]